# EUROPEAN PATENT APPLICATION

(11) **EP 1 619 257 A1**
(43) Date of publication of application: **25.01.2006**
(21) Application number: 04103540.3
(22) Date of filing: 23.07.2004
(51) Int. Cl.: C12Q 1/68

(54) **Method for the in vitro diagnosis of non-alcoholic steatohepatitis**

(71) Applicant: One Way Liver Genomics, S.L., 48160 Vizcaya (ES)
(72) Inventor: Mato, José Maria, One Way Liver Genomics S.L., 48160, Derio, Vizcaya (ES)
(74) Representative: Arias Sanz, Juan

(57) **Abstract**

Method for the in vitro diagnosis of Non-Alcoholic Steatohepatitis.
The invention is related to the in vitro diagnosis of non-alcoholic steatohepatitis (NASH), more specifically to the early diagnosis of the predisposition to develop NASH or to confirm the disease, based on the identification of a cluster of discriminative gene markers specific of NASH that we have called the genomic signature or fingerprint of non-alcoholic steatohepatitis. The invention also relates to the assay of the expression of some of the genes comprised in NASH genomic fingerprint encoding for proteins which can be measured in serum.

## Description

### Field of the invention

The invention is related to the in vitro diagnosis of non-alcoholic steatohepatitis (NASH), more specifically to the early diagnosis of the predisposition to develop NASH or to confirm the disease, based on the identification of a cluster of discriminative gene markers specific of NASH that we have called the genomic signature or fingerprint of non-alcoholic steatohepatitis.

### Background of the invention

Non-alcoholic steatohepatitis is a progressive disease of the liver of unknown etiology characterized histologically by fatty acid accumulation, hepatocyte damage and inflammation resembling alcoholic hepatitis (1,2). NASH is a critical stage in the process that spans from hepatic steatosis to cirrhosis and liver failure (3,4). Obesity and type-2 diabetes are associated to NASH (5,6). Since the prevalence of these diseases is increasing (7), the prevalence of NASH is also expected to increase and therefore, this disease has become an emerging public issue in United States (8) as well as in other countries (9).

NASH is thought to arise from the interaction of many different genes and life-style factors. Mitochondrial impairment, oxidative stress and metabolic deregulation, have all been involved in the pathogenesis of steatohepatitis (10-15). In a previous patent (WO2004/055520), the inventors developed a method of diagnosis of NASH by using molecular markers based on proteomic determination of a set of proteins detected in hepatic tissue samples instead of a genomic fingerprint determination, as the method now disclosed in the present invention. Neither those proteins, nor the genes encoding the same, are included in present invention. Nevertheless, within the genes listed in Table 1, forming part of the genomic fingerprint, there are 4 genes *(CYP2A5* -homologous in mouse to human gene *CYP2A6-, PPARG, MTHFR, CD81)* which have been previously associated to liver diseases (20-23). However none of these 4 genes had been identified as an early gene marker of NASH. Moreover, none of these 4 genes by their own, isolated from the rest of 85 genes, can be used as genomic marker for NASH.

As it is true for other complex diseases, the genetic factors contributing to the development of non-alcoholic steatohepatitis may be more readily identified by combining studies in patients with NASH and in animal models of the disease. One of these models is *MAT1A* knockout (MAT1A-KO) mice. These mice spontaneously develop NASH and hepatocellular carcinoma at about 8 and 15 months of age respectively (14,15). *MAT1A* encodes for methionine adenosyltransferase I and III, the main enzymes responsible of S-adenosylmethionine (SAMe) synthesis in liver (16). Earlier studies concluded that patients with liver cirrhosis (17) and alcoholic hepatitis (18) are deficient in SAMe synthesis; and that treatment with SAMe improves survival in patients with alcoholic liver cirrhosis (19).

Early diagnosis of NASH has been held back by the lack of reliable early markers of non-alcoholic steatohepatitis development. To identify genes that are early markers of NASH we analyzed the data generated from genome-wide expression profiling of liver samples from patients with NASH and from *MAT1A* knockout (MAT1A-KO) mice.

### Summary of the invention

Here we analyzed the data generated from genome-wide expression profiling (microarray data) of liver samples from patients with NASH and from a mouse model of non-alcoholic steatohepatitis (MAT1A-KO) to obtain the genomic signature of NASH. An analysis technique that combines bioinformatic and statistical methods has been used. This genomic signature of NASH accurately distinguishes between patients with early-stage NASH and healthy controls. Patients with liver steatosis have a genomic signature intermediate between that of patients with NASH and controls, offering the prospect of early diagnosis, which improves the chance to intervene with an effective treatment.

One of the embodiments of present invention is, therefore, a method of diagnosis *in vitro* of NASH in liver tissue samples extracted by biopsy, based in the co-expression of a cluster of 85 genes which shows specific differences gene expression between patients and reference controls. The method allows the easy and reliable evaluation of the potential risk of a "subject" to develop NASH, meaning that allows the determination of those "subjects" that, within a group of population show a risk of developing NASH. The mentioned "subject" may be a subject who has not previously diagnosed with NASH or a subject who has been diagnosed with NASH to be confirmed. As an example, a subject who has not been previously diagnosed with NASH or who has no symptoms may be analyzed in order to obtain information about the possibility of that subject of developing NASH in the future. Therefore, in a particular embodiment, the method allows the evaluation of the predisposition (early diagnosis) of a subject to develop NASH, while in another particular embodiment, the same method allows the confirmation diagnosis of the existence of NASH in a subject. The term "subject" should be interpreted in its broadest meaning: as any mammal which may develop NASH. As an example, some primates as baboons, also develop NASH.

Other embodiment of the invention is a method of diagnosis *in vitro* of steatosis in liver tissue samples extracted by biopsy, based in the co-expression of a cluster of 85 genes that shows specific expression differences between patients and reference controls. The diagnosis of steatosis serves as prognosis or to ascertain the susceptibility of developing NASH in the future of subjects without NASH symptoms at present.

Accordingly to the aforesaid embodiments, the method is based in the co-expression of a cluster or 85 genes, or combinations thereof. Therefore, the genomic fingerprint of NASH is established on the expression of the whole of a set of genes or combinations thereof. By combinations are meant genomic fingerprints or signatures based on the co-expression of groups or sets of any of the 85 genes included in the cluster.

A third embodiment of the invention is based on the fact that some of the 85 genes of the genomic fingerprint encodes for membrane or secreted proteins, the latter may be detected in serum. Accordingly, it is also a goal of the invention, the assay of the proteins encoded by some of the genes intervening in the genomic fmgerprint by conventional methods. Once a gene was characterized by determining its DNA sequence, once it was discovered that the gene expression is altered in patients suffering a specific disease and, once it was determined that the gene encodes for a serum protein, the sequence characterization of the said protein and the development of antibodies against it, is a matter of laboratory routine for a man skilled in the art. The invention would cover, therefore, not only the methods for *in vitro* diagnosis of NASH based in a genomic fingerprint or signature and/or the assay of serum proteins encoded by the genes intervening in said genomic fmgerprint, but also any diagnosis assay kits developed with those purposes and based in the different gene expression of certain specific gene markers (comprised in the 85 genes cluster shown in Table 1) detected in NASH patients when compared with reference-control expression patterns.

### Brief description of the figures

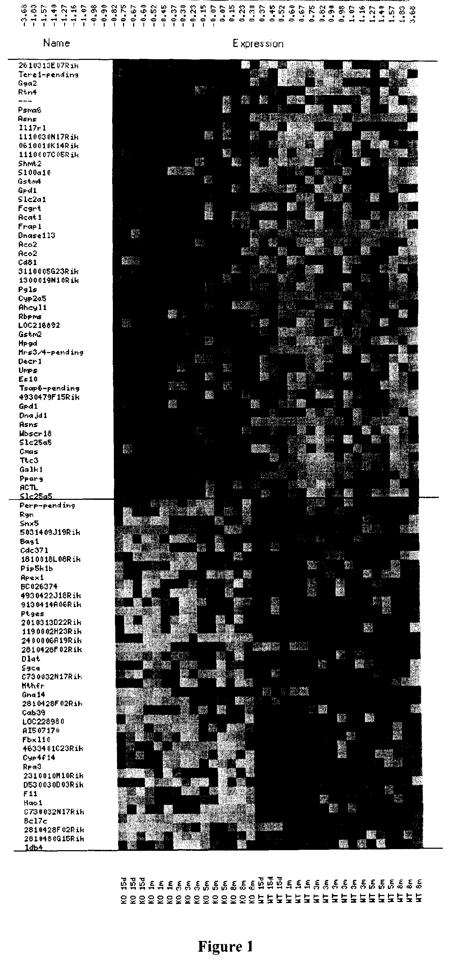

**Figure 1.** Liver expression of the 88 Affymetrix mouse probes, corresponding to the 85 validated early gene markers of non-alcoholic steatohepatitis, in MAT1A knockout and wild-type mice. Black-grey tones indicate differential expression (grey, up-regulation; light grey, down-regulation; black, no expression change). Shown values are the difference between expression levels and mean expression level measured in standard deviations. Name, gene name; KO, MAT1A knockout mice; WT, wild-type mice. The age of the mouse is indicated in days (d) or months (m).

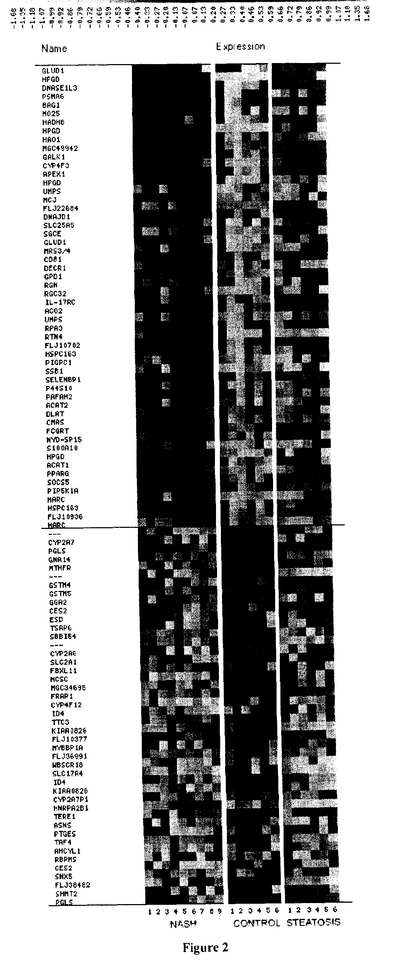

**Figure 2.** Liver expression of the 98 Affymetrix human probes, corresponding to the 81 validated early gene markers of non-alcoholic steatohepatitis, in NASH patients, healthy controls and patients with hepatic steatosis. Black-grey tones indicate differential expression (grey, up-regulation; light grey, down-regulation; black, no expression change). Shown values are the difference between expression levels and mean expression level measured in standard deviations. Name, gene name; NASH, patients with non-alcoholic steatohepatitis; Control, healthy controls; Steatosis, patients with hepatic steatosis.

### Detailed description of the invention

We first analyzed gene expression profiles of liver samples from MAT1A-KO and wild-type (WT) mice at 15 days, 1, 3, 5 and 8 months of age using the MOE430A Affymetrix chip containing 22690 entries. We used two statistical filters to select gene markers of NASH. Genes were first analyzed statistically using an ANOVA type I test (http://www.uv.es/~lejarza/anova/anova.html) and 3508 genes passed this first test (p<0.025). As it is known, this test shows which genes behave coherently within each class. This test is useful to remove genes whose profile varies because reasons other than age or type of mouse. The classes in which the genes were divided were 10 (WT 15 days, 1, 3, 5 and 8 months; and KO 15 days, 1, 3, 5 and 8 months). Genes were then statistically analyzed using a t-test (http://home.clara.net/sisa/t-thlp.htm) and 3266 genes passed this second test (p<0.025). This test selects genes that have an average expression significantly different between WT and KO mice not taking into account the age of the mice. Matching these two sets of genes identified a group of 1496 common genes to both tests that are genes that have different expression between WT and KO mice and behave coherently within each group.

To validate which of these mouse genes were also gene markers of non-alcoholic steatohepatitis in humans, we examined gene expression profiles of liver samples from 6 subjects with normal liver function and 9 patients with NASH grade 1 using the HG U133 Plus 2 Affymetrix chip containing 54675 entries. Statistical analysis of these two sets of human samples using a t-test (p<0.05) identified a group of 4272 discriminative human genes (4679 Affymetrix probes) corresponding to 1700 homolog mouse genes (2556 Affymetrix mouse probes). Matching this set of 1700 genes with the 1496 discriminative genes identified above as gene markers of NASH in mouse revealed a set of 218 common gene markers of NASH to human and mouse.

Finally, to validate which of this set of genes are early gene markers of NASH, we examined gene expression profiles of liver tissues from 15 and 30 days old WT and KO mice. This comparison allows to select genes that have an average expression significantly different between WT and KO mice early in the development of NASH. To perform that comparison statistical analysis of these two sets of samples, a t-test with a p<0.025 was used. By means of that t-test a group of 1346 discriminative genes was identified. Matching this set of genes with the 218 common gene markers of NASH in human and mouse identified above revealed a set of 85 discriminative genes (Table 1) that fulfill the following four criteria: are associated to NASH both in human and mouse, are early gene markers of NASH, remain differentially expressed during development of NASH, and their variance is small during development of the disease. This set of genes, that we have termed the genomic signature of non-alcoholic steatohepatitis is shown in Table 1. Using this signature of NASH we could group MAT1A-KOs with MAT1A-KOs, WT mice with WT mice (Fig. 1), NASH patients with NASH patients, and healthy controls with healthy controls (Fig. 2). Patients with liver steatosis have a genomic signature intermediate between that of patients with non-alcoholic steatohepatitis and healthy controls, with some patients resembling more healthy controls and others resembling more NASH patients (Fig. 2) suggesting that this signature may be useful to identify patients at high risk for the development of non-alcoholic steatohepatitis.

**Table 1 Gene cluster constituting NASH genomic fmgerprint**

| NAME | **DESCRIPTION** |
|---|---|
| Ces2 | Similar to carboxylesterase 2 (intestine, liver) Cytochrome P450, subfamily IVF, polypeptide 14 |
| CYP4F12 | (leukotriene B4 omega hydroxylase) |
| CYP4F3 | Cytochrome P450, subfamily IVF, polypeptide 14 (leukotriene B4 omega hydroxylase) |
| Dnase113 | Deoxyribonuclease 1-like 3 |
| FLJ22684 | RIKEN cDNA 5031409J19 gene |
| Fcgrt | Fc receptor, IgG, alpha chain transporter |
| PIGPC1 | P53 apoptosis effector related to Pmp22 |
| Ptges | Prostaglandin E synthase |
| Sgce | Sarcoglycan, epsilon |
| Cd81 | CD 81 antigen Solute carrier family 2 |
| S1c2a1 | (facilitated glucose transporter), member 1 |
| Acat1 | Similar to Acetyl-Co A acetyltransferase 1 |
| Aco2 | Similar to Aconitase 2, mitochondrial |
| Decr1 | 2,4-dienoyl CoA reductase 1, mitochondrial |
| Dlat HADHB | Similar to Dihydrolipoamide S-acetyltransferase (E2 component of pyruvate dehydrogenase complex) Similar to hydroxyacyl-Coenzyme A dehydrogenase3-ketoacyl-Coenzyme A thiolaseenoyl-Coenzyme A hydratase, beta subunit |
| MCSC | Similar to mitochondrial Ca2+-dependent solute carrier |
| Mrs3/4 | Similar to putative mitochondrial solute carrier |
| Shmt2 | Similar to serine hydroxymethyltransferase 2 (mitochondrial) |
| S1c25a5 | Solute carrier family 25 (mitochondrial carrier; adenine nucleotide translocator) |
| ACAT2 | Acetyl CoA transferase-like protein |
| Asns | Asparagine synthetase |
| Bag1 | Bcl2-associated athanogene 1 |
| Fbx11 | F-box protein FBL11 |
| FLJ10377 | Expressed sequence AI503051 |
| GLUD1 | Glutamate dehydrogenase 1 |
| Galk1 | Galactokinase |
| Gga2 | Homolog to ADP-ribosylation factor binding protein GGA2 (Gamma-adaptin related protein 2) |
| Gpd1 | Glycerol phosphate dehydrogenase 1, cytoplasmic adult |
| Gstm4 | Glutathione transferase GSTM7-7 |
| HARC | Cell division cycle 37 homolog (S. cerevisiae)-like |
| HNRPA2B 1 | Heterogeneous nuclear ribonucleoprotein A2B1 |
| Id4 | Inhibitor of DNA binding 4 |
| IL-17rC | Interleukin 17 receptor-like |
| MGC49942 | Myb-like DNA-binding domain containing protein |
| MO25 | Calcium binding protein, 39 |
| Mthfr | Similar to 5,10-methylenetetrahydrofolate reductase (NADPH) |
| MYBBP1A | MYB binding protein (P160) 1a |
| Rgn | Regucalcin |
| Rtn4 | Homolog to GLUT4 VESICLE 20 KDA PROTEIN |
| SSB-1 | Similar to SPRY domain-containing SOCS box protein SSB-1 SSB-1 |
| TAF4 | TAF4A RNA polymerase II, TATA box binding protein (TBP)-associated factor 135 kDa |
| Tere1 | Similar to transitional epithelia response protein |
| Tsap6 | Similar to Dudulin 2 |
| Ahcy11 | S-adenosylhomocysteine hydrolase-like 1 |
| Apex1 | Apurinicapyrimidinic endonuclease |
| Cmas | CMP-N-acetylneuraminic acid synthetase |
| Cyp2A6 | Cytochrome P450 |
| Cyo2A7 | Cytochrome P450 |
| Cyp2A7P1 | Cytochrome P450 |
| ESD | Esterase 10 |
| F11 | Coagulation factor XI |
| FLJ10702 | Similar to ADP-ribosylation factor-like 10C |
| FLJ36991 | Similar to Zinc finger protein 565 |
| Frap1 | FK506 binding protein 12-rapamycin associated protein 1 |
| Gna14 | Guanine nucleotide binding protein, alpha 14 |
| Gstm5 | Glutathione S-transferase, mu 5 |
| Hao1 | Hydroxyacid oxidase 1, liver |
| Hpgd | Similar to hydroxyprostaglandin dehydrogenase 15-(NAD) |
| MCSC | Similar to mitochondrial Ca2+-dependent solute carrier |
| MGC34695 | Clone:1300019N10, hypothetical protein |
| NYD-SP15 | Expressed sequence AI449174 |
| PAFAH2 | Similar to platelet-activating factor acetylhydrolase 2, 40kDa |
| Pgls | Similar to 6-phosphogluconolactonase |
| Pip5k1A Pip5k1A | Similar to phosphatidylinositol-4-phosphate 5-kinase, type 1 beta |
| Pparg | Peroxisome proliferator activated receptor gamma |
| Psma6 | Proteasome (prosome, macropain) subunit, alpha type 6 |
| Rbpms | RNA binding protein gene with multiple splicing |
| RGC32 | Similar to response gene to complement 32 |
| S100a10 | S100 calcium binding protein A10 (calpactin) |
| SLC17A4 | Similar to solute carrier family 17 (sodium phosphate), member 2 |
| Snx5 | Sorting nexin 5 |
| Umps | Uridine monophosphate synthetase |
| Wbscr18 | Similar to syntaxin 1A (brain) |
| SELENBP | Selenium binding protein |
| Dnajd1 / MJC | DnaJ (Hsp40) homolog, subfamily D, member 1 |
| FLJ10936 | Clone:8430405J03, homolog to CDNA FLJ10936 |
| FLJ38482 | Similar to RIKEN cDNA 3110005G23 gene, MGC:28055 |
| HSPC163 | Similar to HSPC163 protein |
| KIAA0826 | RIKEN cDNA 2510002A14 gene |
| P44S10 | Similar to proteasome regulatory particle subunit P44S10 |
| Rpa3 | Similar to replication protein A3, 14kDa |
| SBB154 | Similar to hypothetical transmembrane protein SBBI54 |
| SOCS-5 | Socs-5 |
| Ttc3 | Tetratricopeptide repeat domain |

Among the genes comprising the signature of non-alcoholic steatohepatitis, 34, more than would be expected by chance, were enzymes, the majority being hydrolases, transferases, and oxidoreductases (Table 1). The list in Table 1 also includes several ligand-binding genes (heavy metal binding, nucleotide binding, protein binding, receptors and transcription factors); transporters (carbohydrate, electron transporter, and protein transporters); apoptosis regulators; chaperones; blood coagulation factors; and several genes with unknown function. However, all the genes comprised in the cluster constituting the NASH fmgerprint or signature, even those with unknown function, have value as integrated diagnosis tool for the disease, by measuring the co-expression of the genes comprised in the cluster or combinations thereof, in patients as compared with the reference gene co-expression in control subjects.

Commercially available genechips or biochips expression array developed by Affymetrix, has been initially used for assaying gene expression of the cluster of 85 genes conforming the genomic fmgerprint of NASH. However, any other gene expression technology would be suitable for assaying that gene expression according to the invention.

The basic concept behind the use of genechips arrays for gene expression is simple: labeled cDNA or cRNA targets derived from the mRNA of an experimental sample are hybridized to nucleic acid probes attached to the solid support. By monitoring the amount of label associated with each DNA location, it is possible to infer the abundance of each mRNA species represented. Although hybridization has been used for decades to detect and quantify nucleic acids, the combination of the miniaturization of the technology and the large and growing amounts of sequence information, have enormously expanded the scale at which gene expression can be studied.

Probe selection and array design lie at the heart of the reliability, sensitivity, specificity, and versatility of genechips expression probe arrays. Because genechips manufacturing technology synthesizes probes with defined sequences, positions, and lengths, array performance can be optimized using data collected from multiple databases, bioinformatics tools, and experiment-trained computer models.

Some of the key elements of selection and design are common to the production of all genechips arrays, regardless of their intended application. Strategies to optimize probe hybridization, for example, are invariably included in the process of probe selection. Hybridization under particular pH, salt, and temperature conditions can be optimized by taking into account melting temperatures and using empirical rules that correlate with desired hybridization behaviours. Based on data from thousands of experiments that monitor the hybridization of target sequences in complex mixtures, Affymetrix uses computer models to predict the intensity and concentration-dependence of probe hybridization. Thus, data quality can be maximized, while probe number is minimized.

A core element of array design, the Perfect Match/Mismatch probe strategy, is also universally applied to the production of genechips arrays. For each probe designed to be perfectly complementary to a target sequence, a partner probe is generated that is identical except for a single base mismatch in its center.

These probe pairs, called the Perfect Match probe (PM) and the Mismatch probe (MM), allow the quantitation and subtraction of signals caused by non-specific cross-hybridization. The difference in hybridization signals between the partners, as well as their intensity ratios, serve as indicators of specific target abundance.

Several aspects of probe selection and array design are dictated by an array's intended use. To select probes for gene expression arrays, sequence and annotation data from multiple databases are integrated. Affymetrix manufactures arrays to monitor the global activities of genes in yeast, *Arabidopsis, Drosophila,* mice, rats, and humans. For human arrays, expressed sequences from databases-including GenBank®, RefSeq, and dbEST, are collected and clustered into groups of similar sequences. Using clusters provided by the UniGene database as a starting point, sequences are further subdivided into subclusters representing distinct transcripts.

This categorization process involves alignment to the human genome, which reveals splicing and polyadenylation variants. The alignment also extends the annotation information supplied by the databases pinpointing low quality sequences. These can be trimmed for the subsequent generation of high quality consensus sequences. Alternatively, quality ranking can be used to select representative sequences, called exemplars, for probe design.

Before selecting individual probes from either exemplars or consensus sequences, the 5' to 3' orientation of each transcript must be determined. Affymetrix uses computer algorithms that combine information from public annotations with in-house identification of splice signals, polyadenylation sites, and polyadenylation signals to distinguish sense from antisense strands. If the orientation cannot be determined unequivocally due to contradictory information, then the probes for both strands are generated.

In general, 11 to16 probes are selected among all possible 25-mers to represent each transcript. In addition to choosing the probes based on their predicted hybridization properties, candidate sequences are filtered for specificity. Their potential for cross-hybridizing with similar, but unrelated sequences, is evaluated.

To obtain a complete picture of a gene's activity, some probes are selected from regions shared by multiple splice or polyadenylation variants. In other cases, unique probes that distinguish between variants are favored. Inter-probe distance is also factored into the selection process. Probes are 3'-biased to match the target generation characteristics of our sample amplification method, but they are also widely spaced to sample various regions of each transcript and provide robustness of detection.

Quality control applications are used to monitor every step in the process of probe selection and array design. In addition, the continuous updating of sequence information and the development of new algorithms to mine databases and predict hybridization behaviors ensures a high quality solution.

Using technologies adapted from the semiconductor industry, genechips manufacturing begins with a 5-inch square quartz wafer. Initially the quartz is washed to ensure uniform hydroxylation across its surface. Because quartz is naturally hydroxylated, it provides an excellent substrate for the attachment of chemicals, such as linker molecules, that are later used to position the probes on the arrays.

The wafer is placed in a bath of silane, which reacts with the hydroxyl groups of the quartz, and forms a matrix of covalently linked molecules. The distance between these silane molecules determines the probes' packing density, allowing arrays to hold over 500,000 probe locations, or features, within a mere 1.28 square centimeters. Each of these features harbors millions of identical DNA molecules. The silane film provides a uniform hydroxyl density to initiate probe assembly. Linker molecules, attached to the silane matrix, provide a surface that may be spatially activated by light.

Probe synthesis occurs in parallel, resulting in the addition of an A, C, T, or G nucleotide to multiple growing chains simultaneously. To define which oligonucleotide chains will receive a nucleotide in each step, photolithographic masks, carrying 18 to 20 square micron windows that correspond to the dimensions of individual features, are placed over the coated wafer. The windows are distributed over the mask based on the desired sequence of each probe. When ultraviolet light is shone over the mask in the first step of synthesis, the exposed linkers become deprotected and are available for nucleotide coupling. Critical to this step is the precise alignment of the mask with the wafer before each synthesis step. To ensure that this critical step is accurately completed, chrome marks on the wafer and on the mask are perfectly aligned.

Once the desired features have been activated, a solution containing a single type of deoxynucleotide with a removable protection group is flushed over the wafer's surface. The nucleotide attaches to the activated linkers, initiating the synthesis process.

Although the process is highly efficient, some activated molecules fail to attach the new nucleotide. To prevent these "outliers" from becoming probes with missing nucleotides, a capping step is used to truncate them. In addition, the side chains of the nucleotides are protected to prevent the formation of branched oligonucleotides.

In the following synthesis step, another mask is placed over the wafer to allow the next round of deprotection and coupling. The process is repeated until the probes reach their full length, usually 25 nucleotides.

Although each position in the sequence of an oligonucleotide can be occupied by 1 of 4nucleotides, resulting in an apparent need for 25 x 4, or 100, different masks per wafer, the synthesis process can be designed to significantly reduce this requirement. Algorithms that help minimize mask usage calculate how to best coordinate probe growth by adjusting synthesis rates of individual probes and identifying situations when the same mask can be used multiple times.

Once the synthesis is complete, the wafers are deprotected, diced, and the resulting individual arrays are packaged in flowcell cartridges. Depending on the number of probe features per array, a single wafer can yield between 49 and 400 arrays.

The manufacturing process ends with a comprehensive series of quality control tests. Additionally, a sampling of arrays from every wafer is used to test the batch by running control hybridizations. A quantitative test of hybridization is also performed using standardized control probes.

Monitoring gene expression lies at the heart of a wide variety of medical and biological research projects, including classifying diseases, understanding basic biological processes, and identifying new drug targets. Until recently, comparing expression levels across different tissues or cells was limited to tracking one or a few genes at a time. Using genechip type arrays, it is possible to simultaneously monitor the activities of thousands of genes.

Although the previously described commercial microarray type has been the one used in present invention for assaying gene expression of the cluster of 85 genes involved in NASH any other gene expression assay may be used. As way of example, instead of probes for human or mouse genes, as used in Affymetrix technology, the same oligonucleotides specifically used in Affimetrix microarray or any other designed to specifically hybridise with segments of the sequences of any of the 85 genes conforming the genomic fingerprint of NASH may also be used. Those oligonucleotides would be linked (printed) to any kind of support, particularly a crystal plate dopped with aminosilane groups, using DMSO as printing buffer. Thus an alternative biochip, achieving also the assay of gene expression in an analogous manner to the genechips developed by Affimetrix, may be used too. Design of oligonucleotides which hybridise with specific sequence fragments of any of the 85 genes comprised in the cluster constituting the NASH signature or fmgerprint it is also a matter for laboratory routine for a man skilled in the art pertaining to the biotechnology field. Oligonucleotide printing may be achieved by a spotter where temperature and humidity are continuously controlled. After oligonucleotide printing the crystal plates are submitted to ultraviolet irradiation. Then cRNA labelled samples are applied to the crystal plates by means, for example, of a Hamilton micropipette a hybridisation takes places during at least 1 h (Amersahm Biosciences). Once hybridisation was completed the plates are washed 3 times and dried out. The crystal plates are then scanned for the label signal coupled to the cRNA, and the amount of labelling calculated by the scanner software indicates the level of gene co-expression existing in each sample for the cluster of genes comprised in the NASH genomic fingerprint or signature.

Another alternative that is envisaged in the invention would be the assay of the gene expression attributed to the cluster of 85 genes conforming the genomic fmgerprint of NASH, also by using oligonucleotides specifically designed to sequence segments of each gene involved in said genomic signature, by means of Polymerase chain reaction (PCR) technique.

Among the genes identified as early markers of non-alcoholic steatohepatitis, 10 encoded for proteins localized in mitochondria, an organelle that has been linked previously to the pathogenesis of NASH by a variety of biochemical experiments (10-12), 4 were for proteins localized in the plasma membrane (CD81; SLC2A1; PIGPC1; SGCE), and 9 were for proteins that are potentially secreted from the liver (CES2; CYP4F12; CYP4F3; DNASE1L3; FLJ22684; FCGRT; PIGPC1; PTGES y SGCE). These last two groups of genes are of particular interest since proteins that are secreted from cells into the extracellular space or are at the plasma membrane represent the major class of molecules involved in intercellular communication and have additional interest as potential serum markers for early diagnosis of NASH development.

In order to detect said encoded proteins in serum, commercial antibodies should be used or, in absence of antibodies available in the market against any of the secreted proteins, they would be produced also by standard methods. Specificity of both, commercially or newly developed antibodies would be tested by Western blot techniques.

In case that antibody production would be necessary, we shall seek antigen sequences of the proteins using public programs (29). For each marker identified, we shall select 3 immunogenic peptides to generate 3 polyclonal (in rabbit) antibodies (Ab) against the molecular target identified. Regardless of the origin of the antibody, commercial or new production, side by side we shall clone and express each one of the markers in the BL21 (D3) prokaryote system: the expression of the cloned marker gene in a vector inherent to the BL21 system is induced thanks to the presence of a copy of the T7 RNA polymerase gene under control of the lac UV5 promoter, which is induced by IPTG (30). The versatility and efficacy of this prokaryote system assures levels of expression sufficient to enable each recombinant protein to be utilized as control in Western blot to confirm the greater or lesser specificity of the three synthesized Ab against the molecular target. We shall check by means of ELISA the capacity of the Ab for union selected to the antigen (target), utilizing as controls, amongst others, the nonconjugated peptide, the pre-immune serum and the recombinant protein of the marker. This sequence of controls will allow the determination of the Ab with greater specificity that will be valid in control sera and patients with NASH, by means of ELISA.

By an immunoassay, preferentially enzyme linked immuno assay (ELISA), the sensitivity of the serum protein detection method would be measured. Enzyme-linked Immunosorbent Assays (ELISAs) combines the specificity of antibodies with the sensitivity of simple enzyme assays, by using antibodies or antigens coupled to an easily- assayed enzyme. ELISAs can provide a useful measurement of antigen or antibody concentration. There are two main variations on this method: The ELISA can be used to detect the presence of antigens that are recognized by an antibody or it can be used to test for antibodies that recognize an antigen. An ELISA is a five-step procedure: 1) coat the microtiter plate wells with antigen; 2) block all unbound sites to prevent false positive results; 3) add antibody to the wells; 4) add anti-mouse IgG conjugated to an enzyme; 5) reaction of a substrate with the enzyme to produce a colored product, thus indicating a positive reaction. There are many different types of ELISAs which can be used in the invention but also other immunoassays based on specific antibodies against any of the secreted proteins, or combinations thereof, encoded by any of the genes forming the genomic fingerprint of NASH disease, as disclosed in the invention, may be suitable to carry out the required detection of the aforesaid proteins in the serum of patients suffering from NASH or with a high risk or predisposition to develop NASH. At this stage we will use polyclonal antibodies against the serum proteins codified by those up-regulated genes which are part of the genomic signature for NASH: Cyp4f3, DNASEIL3, FLJ22684, FCGRT, PIGPC1, SGCE and CD81. Nevertheless, also proteins encoded for genes comprised in NASH genomic fingerprint, being down-regulated (PTGES, CES2, CYP4F12 and SLC2A1) may be useful serum markers when compared with control serum values measured in healthy subjects. Moreover, also any other type of antibodies may be used in the present invention, comprising: monoclonal antibodies, recombinant fragments of antibodies, etc...

There are many different types of ELISAs, which can detect the presence of protein in serum or supernatant. One of the most common types of ELISA is the so-called "sandwich ELISA." It is termed this because the antibody that you are detecting gets sandwiched between an antigen and a chromogenically-conjugated antibody. Below you will find a basic protocol for this assay:
· To coat the plate with the appropriate antigen, fill the microwells of a Nunc Maxi-Sorp Immuno Plate with 100uL of the diluted antigen.
· Incubate at 4°C overnight
· Wash the unbound antigen off the plate by flicking the contents of the plate into the sink, fill the wells with DI water, flick again, repeat 2X with PBS-Triton.
· Block non-specific binding by adding 200uL of 1%BSA/PBS
· Incubate for 30-60minutes at Room Temperature (RT)
· Wash plate as above
· Add 100uL of (diluted) samples to appropriate wells. Be sure to include positive and negative controls, and, if necessary, a standard curve.
• Incubate for 1 hour at RT
• Repeat washing step
• Prepare appropriate dilution of the second step antibody conjugated either with Alkaline Phosphatase or Horseradish Peroxidase. (antibodies should be titrated for optimal dilution)
• Add 100uL of second step antibody to wells and incubate for 1hr
• Repeat washing step
• Prepare substrate solution*
• Add 100uL of substrate to well and incubate at RT for 60min
• add stopping solution if appropriate
• Read plates on an ELISA plate reader*
   *Common Substrates and the appropriate plate reader setting
• ABTS: 405-410 nm
• TMB: non-stopped 620-650 nm, stopped 450 nm
• OPD: non-stopped 450 nm, stopped 490 nm
• pNPP: 405-410 nm
• BluePhosTM: 595-650 nm

### Example 1: Selection of Patients

Gene expression was determined in liver biopsies from 3 groups: 1) nine patients diagnosed of early-stage steatohepatitis (7 female, 2 male, mean age 41.1 years, range 24 - 61 years). A diagnosis of NASH grade 1 (25) was established histologically (macrovesicular steatosis, lobular, portal inflammation and Mallory bodies) in the absence of other (viral, alcohol, metabolic) causes of non-alcoholic steatohepatitis; 2) six patients diagnosed of non-alcoholic hepatic steatosis (4 female, 2 male, mean age 43.3 years, range 23 - 72 years,). A diagnosis of non-alcoholic hepatic steatosis was established histologically (25); and 3) six healthy control subjects (3 female, 3 male, mean age 57.6 years, range 23 -79 years) with normal liver histology.

Liver biopsies were divided into two, one was processed for routine histology and the other was immediately frozen in liquid nitrogen and stored at -80°C for subsequent RNA isolation and measurement of gene expression.

### Example 2: Animal Experiments

Gene expression was determined in liver samples from male MAT1A-KO homozygous and wild-type littermates fed a standard diet. Liver samples were obtained fifteen-days, one-, three-, five- and eight-months after birth. Liver specimens were snap frozen for gene expression analysis and stored at -80°C. At least three independent liver samples were used for RNA isolation and measurement of gene expression for each age and condition.

### Example 3: RNA extraction, target preparation, array hybridization and scanning

The RNA samples were processed as recommended by Affymetrix (Santa Clara, CA). Briefly, after column cleanup of the RNA [Qiagen Rneasy, (Chatsworth, CA)], cDNA was synthesized by using an oligo-dT primer (Superscript choice system; Life Technology) attached to a sequence of the T7 promoter region. This cDNA was then used to perform *in vitro* transcription by using a bacteriophage T7RNA polymerase promoter and incorporating biotin-labeled nucleotides [IVT Labeling kit from Affymetrix, Santa Clara, CA]. Labeled cRNA was purified, quantified spectrophotometrically, fragmented and hybridized to the Affymetrix Mouse Genome MOE430A (mouse samples) or the Affymetrix Human Genome U133 Plus 2.0 (human samples). The arrays were washed, stained, and scanned by using a confocal scanner (Affymetrix). Finally, the results were analyzed by using the Genechips Operating Software (GCOS) Version 1.1 produced by Affymetrix.

### Example 4: Data Analysis

Expression signals for each microarray where obtained using the Affymetrix MAS 5.0 software. These signals where normalized for each entry. Hence, fmal expression level is the difference from the mean expression of the signal for each gene measured in standard deviations. In addition, a nonlinear transformation (arcsinh) was applied to these values to diminish the effect of outliers. Analysis and clustering of the gene expression data thus generated was performed with GARBAN (http://www.garban.org) (26). This system is implemented with bioinformatic tools that organize large amount of gene expression data into biological relevant clusters and classify them according to the ontological criteria of the Gene Ontology Consortium (27) (http://www.geneontology.org/external2go/tigr2go) and Gene Atlas (28) (http://www.citi2fr/GENATLAS/welcome.htlm) databases and other public sources.

### References

1. Reid AE. Nonalcoholic steatohepatitis. Gastroenterology **121,**710-723 (2001).
2. Brunt EM. Nonalcoholic steatohepatitis: defmition and pathology. Semin Liver Dis. 21,3-16 (2001).
3. Teli M, James OF, Burt AD, Bennett MK, day CP. A natural history of nonalcoholic fatty liver: a follow-up study. Hepatology 22,1714-1717 (1995).
4. Angulo P. Nonalcoholic fatty liver disease. N Engl J Med 346,1221-1231 (2002).
5. Wanless IR, Lentz JS. Fatty liver hepatitis (steatohepatitis) and obesity: an autopsy study with analysis of risk factors. Hepatology 12,1106-1110 (1990).
6. Marchesini G, Brizi M, Morselli-Labate AM, Bianchi G, Bugianesi E, McCullough AJ, Forlani G, et al. Association of nonalcoholic fatty liver disease with insulin resistance. Am J Med 107,450-455 (1999).
7. Flegal KM, Carroll MD, Kuczmarski RJ, Johnson CL. Overweight and obesity in the United States: prevalence and trends, 1960-1994. Int J Obes Relat Metab Disord 22,39-47 (1998).
8. McCullough AJ. Update on nonalcoholic fatty liver disease. J Clin Gastroenterol 34,255-262 (2002).
9. Farell GC. Non-alcoholic steatohepatitis: what is it, and why is it important in the Asia-Pacific region? J Gastroenterol Hepatol 18,124-138 (2003).
10. Yang S, Zhu H, Li Y, Lin H, Gabrielson K, Trush MA, Diehl AM. Mitochondrial adaptation to obesity-related oxidant stress. Arch Biochem Biophys 378,259-268 (2000).
11. Perez-Carreras M, Del Hoyo P, Martin MA, Rubio JC, Martin A, Castellano G, Colina F, Arenas J, Solis-Herruzo JA. Defective hepatic mitochondrial respiratory chain in patients with nonalcoholic steatohepatitis. Hepatology 38,999-1007 (2003).
12. Santamaría E, Avila MA, Latasa MU, Rubio A, Martin-Duce A, Lu SC, Mato JM, Corrales FJ. Functional proteomics of nonalcoholic steatohepatitis: Mitochondrial proteins as targets of S-adenosylmethionine. Proc Natl Acad Sci USA **100,**3065-3070 (2002).
13. Li Z, Lin H, Yang S, Diehl AM. Murine leptin deficiency alters Kupffer cell production of cytokines that regulate the innate immune system. Gastroenterology **123**,1304-1310 (2002).
14. Lu SC, Alvarez L, Huang Z-Z, Chen L, Corrales FJ, Avila MA, Kanel, G., Mato, J.M. Methionine adenosyltransferase 1A knockout mice are predisposed to liver injury and exhibit increased expression of genes involved in proliferation. Proc. Natl. Acad. Sci. USA **98**,5560-5565 (2001).
15. Martinez-Chantar ML, Corrales FJ, Martinez-Cruz AL, Garcia-Trevijano ER. Huang Z-Z, Chen L, Kanel G, Avila MA, Mato JM, Lu SC. Spontaneous oxidative stress and liver tumors inm mice lacking methionine adenosyltransferase 1A. FASEB J 2002
   http://WWW.fasebj.org/cgi/doi/10.1096/fj.02-0078fje
16. Mato JM, Corrales FJ, Lu SC, Avila MA. S-Adenosylmethionine: a control switch that regulates liver function. FASEB J **16**,15-26 (2002)
17. Duce AM, Cabrero C, Ortiz P, Mato JM. SAM synthetase activity and phospholipid methyltransferase are inhibited in human liver cirrhosis. Hepatology **8**,65-68 (1988).
18. Lee TD, Sadda MR, Mendler MH, Bottiglieri T, Kanel G, Mato JM, Lu SC. Abnormal hepatic methionine and glutathione metabolism in patients with alcoholic hepatitis. Alcohol Clin Exp Res **28**,173-81 (2004).
19. Mato JM, Cámara J, Fernández de Paz J, Caballeria L, Coll S, Caballero A, Garcia-Buey L, Beltrán J, Benita V, Caballeria J, Solà R, Moreno-Otero R, Barrao F, Martin-Duce A, Correa JA, Parés A, Barrao E, Garcia-Magaz I, Puerta JL, Moreno J, Boissard G, Ortiz P, Rodés J. S-adenosylmethionine in alcoholic liver cirrhosis: A randomized placebo-controlled, double-blind, multicentre trial. J of Hepatology **30**,1081-1089 (1999).
20. Niemela O, Parkkila S, Juvonem RO, Viitala K, Gelboin HV, Pasanen M. Cytochrome P450 2A6, 2E1, and 3A and production of protein-aldehyde adducts in the liver of patients with alcoholic and non-alcoholic liver diseases. J Hepatol 33,893-901 (2000).
21. Everett L, Galli A, Crabb D. The role of hepatic peroxisome proliferators- activated receptors (PPARs) in health and disease. Liver **20,**191-199 (2000).
22. Schwahn BC, Chen Z, Laryea MD, Wendel U, Lussier-Cacan S, Genest J Jr, Mar MH, Zeisel SH, Castro C, Garrow T, Rozen R. Homocysteine-betaine interactions in a murine model of 5,10-methylenetetrahydrofolate reductase deficiency. FASEB J **17**,512-514 (2003).
23. Bataller R, Paik YH, Lindquist JN, Lemasters JJ, Brenner DA. Hepatitis C virus core and nonstructural proteins induce fibrogenic effects in hepatic stellate cells. Gastroenterology **126**,529-540 (2004).
24. Lee HK, Hsu AK, Sajdak J, Qin J, Pavlidis P. Coexpression analysis of human genes across many microarray data sets. Genome Res 14,1085-1094 (2004).
25. Brunt EM, Janney CG, Di Bisceglie AM, Neuschwander-Tetri BA, Bacon BR. Nonalcoholic steatohepatitis (NASH): a proposal for grading and staging the histological lesions. Am J Gastroenterol **94**,2467-2474 (1999).
26. Martínez-Cruz LA, Rubio A, Martinez-Chantar ML, Labarga A, Barrio I, Podhorski A, Segura V, Sevilla JL, Avila MA, Mato JM. Genomic analysis and rapid biological annotation of cDNA microarray and proteomic data. Bioinformatics **19,**2158-60 (2003).
27. The Gene Ontology Consortium. Gene Ontology: tool for the unification of biology. Nature Genet **25**,25-29 (2000).
28. Su AL, Cooke MP, Ching KA, Hakak Y, Walker JR, Wiltshire T, Orth AP, Vega RG, Sapinoso LM, Moqrich A, Patapoutian A, Hampton GM, Schultz PG, Hogenesch JB. Large-scale analysis of the human and mouse transcriptomes. Proc Natl Acad Sci USA **99**,4465-4470 (2002).
29. Kolaskar AS, Tongaonkar PC. A semi-empirical method for prediction of antigenic determinants on protein antigens. FEBS Lett. 1990 Dec **10**;276(1-2):172-4.
30.Alvarez L, Mingorance J, Pajares MA, Mato JM. Expression of rat liver S-adenosylmethionine synthetase in Escherichia coli results in two active oligomeric forms. Biochem J. 1994 Jul 15;**301** (Pt 2):557-61.

## Claims

1. A method for the diagnosis *in vitro* of NASH that comprises:
a) obtaining a sample from a subject;
b) detecting and quantifying in said sample the level of expression of any of the 85 genes shown in Table 1 and combinations thereof; and
c) comparing the results obtained in step b) with reference values for the expression of those same genes in healthy controls.

2. A method according to claim 1 wherein the sample is a liver tissue sample and the gene expression detected and quantified in said sample is the co-expression of the whole cluster of 85 genes comprised in Table 1.

3. A method according to claims 1 and 2, wherein the detection and quantification of the level of expression of the genes are carried out according to the following steps:
a) isolating mRNA from the liver tissue sample of a subject;
b) synthesizing cDNA corresponding to the mRNA isolated;
c) transcribing the cDNA synthesized into a cRNA, by incorporating labeled nucleotides
d) hybridizing the labeled cRNA obtained with probes or oligonucleotides corresponding to specific sequence fragments of each cRNA corresponding to each one of the 85 genes of Table 1;
e) quantifying the hybridizing cRNA;
f) comparing the results with the final expression level of healthy controls.

4. A method according to claim 3, wherein the probes are supported on a microarray.

5. A method according to claim 3 wherein the oligonucleotides are printed on a crystal plate.

6. A method according to claim 3 wherein the oligonucleotides are hybridized to labeled cRNA by PCR.

7. Assay kits for NASH diagnosis comprising probes or oligonucleotides hybridizing with specific sequence fragments of each cRNA corresponding to each one of the 85 genes, or combinations thereof, comprised in Table 1.

8. A method according to claim 1 wherein the tissue sample is a blood sample.

9. A method according to claims 1 and 8 wherein the genes whose expression level is detected and quantified are genes encoding for secreted proteins selected among: CYP4F3, DNASE1L3, FLJ22684, FCGRT, PIGPC1, PTGES, SGCE, CYP4F12, CES2, SLC2A1 and CD81, or combinations thereof.

10. A method according to claim 9, wherein the gene expression is detected and quantified by assaying the concentration in serum of the protein/s encoded by each corresponding gene/s.

11. A method according to claim 10, wherein the detection and quantification of the concentration of the protein/s in serum is carried out by means of the use of specific antibodies against said protein/s.

12. A method according to claim 11, wherein the antibodies are used in an enzyme linked immuno-assay (ELISA).

13. Assay kits for detecting and quantifying any of the proteins, or combinations thereof, encoded by any of the 85 genes comprised in Table 1.

14. Assay kit according to claim 13 wherein the protein detected and quantified is selected among: CYP4F3, DNASE1L3, FLJ22684, FCGRT, PIGPC1, PTGES, SGCE, CYP4F12, SLC2A1, CES2 and CD81 or combinations thereof.

15. Assay kit according to claim 14 wherein the protein is detected by means of a specific antibody.

16. Assay kit according to claim 15 wherein the antibody is coupled to an enzyme linked immuno- assay (ELISA).
